# EUROPÄISCHE PATENTSCHRIFT

(11) **EP 1 693 453 B1**
(45) Veröffentlichungstag und Bekanntmachung des Hinweises auf die Patenterteilung: **25.05.2011**
(21) Anmeldenummer: 05019940.5
(22) Anmeldetag: 13.09.2005
(51) Int. Cl.: C12N 15/10, B01J 20/281

(54) **Verfahren zur Isolierung und Trennung von einzel- und doppelsträngigen Nucleinsäuren sowie Trennsäule zur Durchführung des Verfahrens**
Process for separating single-stranded from double-stranded nucleic acids and the separation column for carrying out this process
Procédé de séparation d'acides nucléiques à un brin et à deux brins ainsi que la colonne de séparation permettant de faire ledit procédé

(30) Priorität: 16.09.2004 DE 102004045332
(43) Veröffentlichungstag der Anmeldung: 23.08.2006
(73) Patentinhaber: Macherey, Nagel GmbH & Co. Handelsgesellschaft, 52355 Düren (DE)
(72) Erfinder: Radmacher, Edmund Dr., 52349 Düren (DE); Möller, Klaus Dr., 52249 Eschweiler (DE); Meusel, Markus Dr., 52146 Würselen (DE); Kirsch, Christoph Dr., 50259 Pulheim (DE)
(74) Vertreter: Paul, Dieter-Alfred

(56) Entgegenhaltungen:
- EP-A- 0 743 950
- WO-A-97/30062

## Beschreibung

Die Erfindung betrifft ein Verfahren zur Isolierung und Trennung von einzel- und doppelsträngigen Nucleinsäuren aus einer diese Nucleinsäuren enthaltenden Probe, wobei die doppelsträngig Nucleinsäure DNA und die einzelsträngige Nucleinsäure RNA ist und die einzel- und doppelsträngigen Nucleinsäuren an ein Trägermaterial gebunden werden, von dem die doppelsträngigen Nucleinsäuren mit einer ersten Elutionslösung und die einzelsträngigen Nucleinsäuren anschließend mit einer zweiten Elutionslösung eluiert werden.

Die Präparation von Nucleinsäuren gewinnt zunehmend an Bedeutung. So werden RNA und DNA in medizinischanalytischen, biochemischen und molekularbiologisch arbeitenden Laboratorien benötigt. Die Anwendungsgebiete reichen über die Gentechnologie, Medizin, Veterinärmedizin, Forensik, Molekularbiologie zur Biochemie sowie von der Grundlagenforschung bis zur angewandten Routinediagnostik.

Die Nucleinsäuren enthaltenden Proben werden üblicherweise in einem ersten Schritt aufgeschlossen. Dies ist Stand der Technik und kann beispielsweise in bekannter Weise mechanisch, chemisch, enzymatisch oder durch Behandlung mit Detergenzien erfolgen. An den Zellaufschluss schließt sich dann die Isolierung der Nucleinsäuren an. Klassische Verfahren sind die Cäsiumehlorid-Dichtegradientenzentrifugation oder die Extraktion mit Phenol. Diese Methoden weisen jedoch eine aufwändige Handhabung auf, sind zeitintensiv oder verwenden toxisches Phenol, was problematisch in der Handhabung ist. Aus diesen Gründen haben sich in den letzten Jahren neue Methoden für die Isolierung von Nucleinsäuren durchgesetzt. Hierzu zählen Techniken auf Basis von Silica- und Anionenaustauschern. Während die Anionenaustauscher nach wie vor den Goldstandard für besonders reine DNA-Präparationen darstellen (etwa für Tranefektionsexperimente), haben sich die Silica-basierten Techniken als einfache und kostengünstige Methoden für eine Vielzahl von Applikationen empfohlen.

Seit den 50er Jahren ist es bekannt, dass DNA in Gegenwart chaotroper Salze reversibel an Silikate bindet (Sanibrock, J. and Russel, D.W., 2001, Molecular Cloning - A Laboratory Manual, 3. Ausgabe, Cold Spring Harbor Laboratory Press, New York, Seiten 1.62 ff.). Die Salze bewirken eine Zerstörung der Hydrathülle der Nucleinsäuren und schaffen eine hydrophobe Mikroumgebung. Unter diesen Bedingungen binden die Nucleinsäuren dann an die Silica-Matrix, während Proteine und andere Kontaminanten nicht binden und ausgewaschen werden wird dann die Bindelösung durch einen Niedrigsalzpuffer oder Wasser ersetzt, findet eine Rehydratisierung der Nucleinsäuren statt, die sich dann bereitwillig von der Silicamatrix lösen und eluiert werden können.

An die Präparation der Nucleinsäuren schließen sich dann in der Regel Folgereaktionen an, wie etwa die Polymerase-Kettenreaktion (PCR), die Sequenzierung, die Ligation oder eine Restriktionsanalyse. Die Folgereaktionen erfordern eine bestimmte Quantität und insbesondere Qualität, in der die Nucleinsäuren vorliegen müssen. Die Nucleinsäuren müssen weitestgehend unversehrt sein, in hoher Reinheit vorliegen und frei von Proteinen und Zellmetaboliten sein.

Üblicherweise sind die Bindebedingungen bei silicabasierten Präparationen so eingestellt, dass möglichst selektiv nur eine Form von Nucleinsäure (entweder einzel- oder doppelsträngige) gebunden wird (vgl. WO 97/30062). Zur selektiven Isolierung genomischer doppelsträngiger DNA werden beispielsweise chaotrope Salze wie etwa Guanidinisothiocyanat, Guanidinthiocyanat, Guanidiniumhydrochlorid, Natriumperchlorat oder Natriumjodid in Konzentrationen von 0,1 bis 10 M und aliphatische Alkohole mit 1 bis 5 Kohlenstoffatomen in Konzentrationen von 1-90 Vol.% eingesetzt. Andere selektive Bindebedingungen, wie Lösungen mit Substanzen, die Erdalkaliionen komplexieren, und die Abwesenheit von Alkoholen sind ebenfalls geeignet und aus der EP 0 743 950 bekannt.

Als mineralische Träger dienen vorzugsweise poröse und nicht-poröse Materialien wie beispielsweise Metalloxide, Silicagele, Glaspartikel, Glasmilch, Quarz, Zeolithe, Diatomeenerde oder Titandioxid mit Partikelgrößen von 0,1 bis 1.000 µm. Werden poröse Träger verwendet, so beträgt die Porengröße vorzugsweise 2 bis 1.000 µm. Das poröse oder nicht-poröse Material kann in Form von losen Schüttungen vorliegen oder als Filterschicht ausgebildet sein. Die Filterschichten können aus Glas, Quarz oder Keramik und/oder einer Membran bestehen. Letztere kann wiederum aus Partikeln und/oder Fasern aus mineralischen Trägern und Geweben ausgebildet sein. Bevorzugt werden beispielsweise Glasfasermembranen als mineralischer Träger eingesetzt. Vorzugsweise werden die Filterschichten in Hohlkörper mit Ein- und Auslassöffnung angeordnet.

Die Diskriminierung zwischen doppelsträngiger DNA und einzelsträngigen Nucleinsäuren, insbesondere RNA, kann durch geeignete Waschlösungen zusätzlich verstärkt werden. Die Elution der gebundenen DNA erfolgt dann unter Niedrigsalzbedingungen oder mit Wasser.

Soll selektiv RNA isoliert werden, so werden durch Einsatz von chaotropen Salzen und Alkohol Bindebedingungen eingestellt, unter denen einzelsträngige Nucleinsäuren quantitativ an den mineralischen Träger binden. Dabei ist es unausweichlich, dass auch ein Teil genomischer DNA gebunden wird. Um diese Kontamination zu beseitigen wird die genomische DNA üblicherweise mittels DNase verdaut. Besonders wichtig bei der Präparation von RNA ist die Inaktivierung von RNase, was bereits durch die Lysebedingungen sichergestellt wird oder durch Additive wie β-Mercaptoethanol noch verstärkt wird. Wie die genomische DNA wird die zuvor gebundene, nun von DNA-Kontaminationen befreite RNA unter Niedrigsalzbedingungen oder vorzugsweise mit RNase-freiem Wasser eluiert.

Für eine Vielzahl von Untersuchungen ist es nun besonders vorteilhaft, sowohl die RNA als auch die DNA aus dem gleichen Untersuchungsmaterial simultan zu isolieren. So können z.B. aus limitierenden Mengen des Materials beide Nucleinsäuren isoliert werden. Die simultane Isolierung erlaubt zudem über die DNA die Untersuchung von Genen und über die RNA deren Expression. Das Finden von Korrelationen zwischen DNA-Sequenzen und der Expression dieser Gene erlaubt z.B. Einsichten in Regulationsprozesse. Von besonderer Bedeutung ist die simultane Isolierung von DNA und RNA insbesondere bei der Untersuchung von Spleißprozessen, ein wichtiger Mechanismus bei der Genregulation.

Ein weit verbreitetes Protokoll für die simultane Isolierung von DNA und RNA orientiert sich an der Methode von Chomzynski (Chomzynski P.: A Reagent for the Single-Step Simultaneous Isolation of RNA, DNA and Proteins from Cell and Tissue Samples, BioTechniques 15, 532-534, 1993). Dabei werden Zellen mit einer monophasischen Lösung aus Guanidinisothiocyanat und Phenol lysiert. Die Zugabe von Chloroform führt zur Ausbildung einer zweiten, organischen Phase, in der sich DNA und Proteine anreichern, während sich die RNA im wässrigen Überstand befindet. Die DNA kann aus der organischen Phase von den Proteinen durch sequentielle Fällung mit Ethanol und Isopropanol abgetrennt werden. Nachteilig bei dieser Methode ist die Kontamination der RNA mit DNA, die eine Nachbehandlung notwendig macht. Nachteilig sind zudem die Präparationsdauer zur simultanen Isolierung von RNA und DNA (im günstigsten Fall 3 Stunden) sowie der arbeitstechnische Aufwand. Ferner ist auch der Umgang mit dem toxischen Phenol problematisch.

Ein alternativer Ansatz ist aus der DE 195 06 887 und EP 0 880 535 (Testkit Invisorb TwinPrep *DNA*/*RNA* der Fa.

Invitek GmbH) bekannt. Bei diesem Verfahren wird genomische DNA in Anwesenheit chaotroper Salze an einen mineralischen Träger gebunden, während die RNA in Lösung bleibt. Aus diesem wird sie anschließend mittels Phenol/Chloroform Extraktion isoliert und mit Isopropanol gefällt. Die gebundene DNA steht nach wasch- und Elutionsschritten für weitere Anwendungen zur Verfügung. Dieses Verfahren erlaubt die weitestgehend DNA-freie Präparation von RNA mit vertretbarem Zeitaufwand (< 2 Stunden), auf die problematischen und arbeiteintensiven Schritte der Extraktionen und Pällungen kann auch hier nicht verzichtet werden.

Gänzlich ohne Flügsig-Flüssigextraktion oder sequentielle Fällungen arbeitet das Verfahren, das in der EP 0 743 950 B1 und US 6,180,778 beschrieben ist. EP 0 743 950 B1 offenbart in den ersten drei Ausführungsformen gemäß Anspruch 1 die selektive Bindung von einzel- oder doppelsträngigen Nucleinsäuren an einen mineralischen Träger. In der ersten Ausführungsform wird selektiv RNA gebunden, wlihrend die DNA im Überstand bleibt und aus diesem in Gegenwart chaotroper Salze und Alkohol an einen zweiten mineralischen Träger gebunden wird. In der zweiten Ausführungsform ist es die DNA, die im ersten Schritt in Gegenwart von Substanzen zur Komplexierung von Erdalkaliionen (z.B. EDTA) und Abwesenheit von Alkoholen an den mineralischen Träger gebunden wird. Die in Lösung verbleibende RNA wird dann unter Zugabe von Alkohol an einen zweiten mineralischen Träger gebunden und weiter prozessiert. Die dritte Ausführungsform beschreibt eine Abwandlung der zweiten, indem auf die Substanzen zur Komplexierung von Erdalkaliionen verzichtet wird und diese durch Benetz-, Wasch- oder Dispergiermittel ersetzt werden. Auch hier verbleibt die RNA im Überstand und wird mit einem zweiten mineralischen Träger gereinigt.

Einen abweichenden Ansatz bietet die vierte Ausführungsform, bei der sowohl DNA als auch RNA zunächst gemeinsam an den mineralischen Träger binden und anschließend selektiv eluiert werden. Zur Elution von DNA wird das Trägermaterial mit einer Lösung reduzierter Ionenstärke und Alkoholkonzentration behandelt. Die RNA verbleibt am Träger, während die DNA abgelöst wird. Diese wird dann zur weiteren Aufreinigung an einen zweiten mineralischen Träger gebunden. Nach weiteren Waschschritten kann die DNA letztlich vom zweiten Träger unter Niedrigealzbedingungen oder mit Wasser eluiert werden und steht dann für Folgeanwendungen zur Verfügung. Die am ersten Träger verbliebene RNA kann analog zur DNA gewaschen und eluiert werden. Schließlich steht auch sie für Folgeapplikationen zur Verfügung.

Alternativ kann nach der vierten Ausführungsform auch zunächst die RNA vom ersten Träger eluiert werden. Dies erfolgt durch Zugabe von Benetz-, Wasch- oder Dispergiermitteln und/oder Einsatz von Substanzen zur Komplexierung von Erdalkaliionen. Die so eluierte RNA muss nun weiter gereinigt werden, indem sie an einen zweiten mineralischen Träger gebunden wird und letztlich nach Waschen und Elution für Folgeapplikationen zur Verfügung steht. Die am ersten mineralischen Träger verbliebene DNA wird ebenfalls gewaschen und schließlich eluiert. Damit steht auch sie für Folgeapplikationen zur Verfügung.

Auch wenn auf Extraktionen und Fällungen verzichtet wird und der Ansatz sehr variabel ist, so ist der Nachteil des Verfahrens nach EP 0 743 950 B1 sicher der sehr arbeitsintensive Ablauf unter Einbeziehung von zwei mineralischen Trägern, insbesondere wenn sowohl DNA als auch RNA isoliert werden sollen.

Alle beschriebenen technischen Lösungen weisen somit einen oder mehrere der folgenden Nachteile auf: sie sind arbeitsintensiv, benötigen sehr lange Präparationszeiten, die isolierten Nucleinsäuren müssen in zusätzlichen Schritten nachgereinigt werden, um für Folgeapplikationen zur Verfügung zu stehen, oder es werden toxische Substanzen, wie etwa Phenol, verwendet.

Die Aufgabe der vorliegenden Erfindung besteht in der Verbesserung des Verfahrens der eingangs genannten Art, also der vierten Ausführungsform nach der EP 0 743 950 B1, in der Weise, daß die Nucleinsäuren nach ihrer Isolierung aus der Probe und anschließender Trennung voneinander so vorliegen, daß eine weitere Aufreinigung für die meisten Folgeanwendungen überflüssig ist und sie somit direkt möglich werden. Eine weitere Aufgabe besteht darin, eine für die Durchführung des Verfahrens besonders geeignete Trennsäule bereitzustellen.

Erfindungsgemäß wird die erste Aufgabe dadurch gelöst, daß die Elution der doppelsträngigen Nucleinsäuren in Gegenwart von mindestens einem solchen zweiwertigen Metallion in einer wenigstens so hohen Konzentration durchgeführt wird, daß bei der Elution der doppelsträngigen Nucleinsäuren keine einzelsträngigen Nucleinsäuren eluiert werden, wobei die erste Elutionslösung ein Niedrigsalzpuffer oder Wasser und die zweite Elutionslösung eine Niedrigsalzlösung oder Wasser ist und wobei das mindestens eine zweiwertige Metallion aus der Gruppe der Erdalkalimetalle ausgewählt wird und dessen Konzentration nicht höher als 200 mM ist.

Der Vorteil des erfindungsgemäßen Verfahrens besteht darin, dass die Nucleinsäuren ohne weitere Reinigung oder Aufarbeitungen direkt für Folgeapplikationen zur Verfügung stehen. Das Verfahren nach der Erfindung ist somit nicht nur einfach und schnell durchzuführen, sondern die isolierten und getrennten Nucleinsäuren, wie DNA und RNA, können auch direkt für die unterschiedlichsten Zwecke verwendet werden.

Das Verfahren nach der Erfindung kann auf alle nucleinsäurehaltigen Proben angewandt werden. Dabei kann es sich um Zellen, Gewebe, Blut, Pflanzenmaterial, Lebensmittel, forensische Proben oder anderes nucleinsäurehaltiges Material, wie z.B. in vitro Transkriptionsansätze oder andere Mischungen aus RNA und DNA, handeln. Dem Verfahren gehen in der Regel, falls Zellen vorliegen, ein Aufschluss und eine Lyse der Zellen voraus. Dabei können, wie aus dem Stand der Technik bekannt, z.B. chemische, enzymatische oder mechanische Mittel zum Einsatz kommen. Ein bevorzugtes Verfahren sieht die Lyse der Zellen mittels chaotroper Salze vor. Eine anschließende Zugabe von Alkohol schafft Bedingungen, unter denen alle Nucleinsäuren an das Trägermaterial binden, z.B. an eine angebotene Silicamatrix, vorzugsweise Silicamembranen. Es ist für den Fachmann jedoch offensichtlich, dass das erfindungsgemäße Verfahren nicht auf diesen Träger beschränkt ist, sondern dass jedes für diesen Zweck bekannte Trägermaterial Verwendung finden kann.

Die doppelsträngigen Nucleinsäuren, insbesondere DNA, werden dann nach dem erfindungsgemäßen Verfahren unter Verwendung der ersten Elutionslösung in Gegenwart von mindestens einem zweiwertigen Metallion vom Trägermaterial eluiert. Dabei wurde überraschenderweise gefunden, dass die Konzentration dieses bzw. dieser zweiwertigen Metallionen gering bis sehr gering gewählt werden kann. Gleichwohl ist gewährleistet, dass nur die doppelsträngigen Nucleinsäuren eluiert werden, während die einzelsträngigen Nucleinsäuren auf dem Trägermaterial verbleiben. Erst bei sehr niedrigen Konzentrationen des zweiwertigen Metallions besteht die Gefahr, dass auch einzelsträngige Nucleinsäure, z.B. RNA, miteluiert wird. Die untere Grenze lässt sich durch einfache Versuche festlegen.

Die Maximalkonzentration des zweiwertigen Metallions sollte 200 mM, vorzugsweise 100 mM, nicht überachreiten. Je geringer die Konzentration des mindestens einen zweiwertigen Metallions ist, umso weniger werden Folgeapplikationen gestört. Dies gilt auch für die im nachfolgenden Schritt eluierten einzelsträngigen Nucleinsäuren. Besonders bevorzugte Konzentrationen sind 5 bis 10 mM. Die so eluierten doppelsträngigen Nucleinsäuren sind z.B. in Form von DNA absolut PCR-tauglich, mit Restriktionsenzymen verdaubar, RNA-frei und weisen eine A_{260/280} Ratio von 1,9 bis 2,3 auf.

Das mindestens eine zweiwertige Metallion kann nicht durch ein oder mehrere einwertige Metallionen ersetzt werden, da mit Ionen wie Na⁺ und Li⁺ eine Elution nur der doppelsträngigen Nucleinsäure erst bei Konzentrationen über 200 mM erfolgt. Bei diesem hohen Konzentrationsbereich können jedoch Folgeapplikationen mit der eluierten doppelsträngigen Nucleinsäure nicht direkt anschließend durchgeführt werden, so dass die Vorteile des Verfahrens nach der Erfindung nicht erreicht werden.

Werden Konzentrationen unter 200 mM eingesetzt, findet eine Co-Elution von einzel- und doppelsträngiger Nucleinsäure statt. Eine Differenzierung ist nicht möglich.

Nach der Erfindung ist ferner vorgesehen, dass das Trägermaterial nach der Bindung der Nucleinsäuren und vor der Elution der doppelsträngigen Nucleinsäuren mit einer Waschlösung behandelt wird. Diese an sich bekannte Maßnahme ist dann zweckmäßig, wenn Kontaminationen oder gegebenenfalls verwendete chaotrope Salze unter Aufrechterhaltung der Bindung der Nucleinsäuren von der Bindematrix entfernt werden sollen.

Die Bereitstellung des mindestens einen zweiwertigen Metallione bei dem Schritt der Elution der doppelsträngigen Nucleinsäure kann auf verschiedene Weise erfolgen, da zur Erzielung der Vorteile des erfindungsgemäßen Verfahrens der Zeitpunkt und/oder die Art der Einbringung des zweiwertigen Metallions nicht ausschlaggebend sind. Das oder die zweiwertigen Metallionen können Bestandteil der ersten Elutionslösung und/oder der Waschlösung sein. Ferner kann das mindestens eine zweiwertige Metallion über eine Matrix oder einen festen Träger, wie z.B. ein Filterpapier oder ähnliches, freigesetzt werden, oder es wird in Form einer weiteren Lösung zugeführt. Es ist aber auch möglich, dass das bzw. die zweiwertigen Metallionen in der Waschlösung, aber nicht in der ersten Elutionslösung vorliegen. Hier kommt es offenbar zu einer Art Verschleppungseffekt, der ausreicht, um die Elution der doppelsträngigen Nucleinsäure mit Wasser oder einem Niederselzpuffer durchzuführen, ohne dass es zu einer gleichzeitigen Elution der einzelsträngigen Nucleinsäure kommt. Der Gehalt an Metallionen in der Waschlösung sollte dabei so hoch sein, dass über den Verschleppungseffekt eine Elution ausschließlich der doppelsträngigen Nucleinsäure erfolgt.

Die Einbringung der zweiwertigen Metallionen erfolgt in Form von Erdalkalimetallionen, insbesondere als Ca²⁺ und/oder Mg²⁺, aber auch als Barium-und/oder Strontiumionen. Die Gegenionen haben keinen bis geringen Einfluß auf das Verfahren nach der Erfindung. Gut geeignet sind MgCl₂ und/oder CaCl vorzugsweise im Konzentrationsbereich 5 bis 10 mM.

Statt in flüssiger Form können die zweiwertigen Metallionen auch in fester Form in das Verfahren eingebracht werden, z.B. in Form von Calciumsulfat. Dieser Feststoff kann in der Trennsäule, der Matrix der Trennsäule oder einem Filterpapier vorgelegt werden. Aufgrund der Wasserlöslichkeit des Calciumsulfats werden die zweiwertigen Ionen beim Auftrag von Flüssigkeit auf die Trennsäule sukzessiv an die Lösungen abgegeben. Sofern ausreichend Calciumsulfat vorgelegt wird, werden die Metallionen durch die die Nucleinsäuren enthaltene Probe und den gegebenenfalls verwendeten Waschpuffer nicht vollständig ausgewaschen, so daß dann bei Aufgabe der ersten Elutionslösung noch genügend Calciumionen an die Elutionslösung abgegeben werden mit der Folge, daß nur doppelsträngige Nucleinsäure eluiert wird. Mittels nachfolgender Waschschritte kann dann das Calciumsulfat entfernt werden, bevor dann im zweiten Elutionsschritt die einzelsträngige Nucleinsäure eluiert wird.

Statt mittels Calciumsulfat können die Metallionen auch durch die Verwendung von Bariumsalz, z.B. Bariumchlorid, bereitgestellt werden. Diese Substanz eignet sich vor allem dann, wenn an die Elutionslösung mit den doppelsträngigen Nucleinsäuren besonders hohe Anforderungen, insbesondere was den Gehalt an zweiwertigen Kationen betrifft, gestellt werden. Diese können nämlich in einer Nachbehandlung nach Elution der doppelsträngigen Nucleinsäuren dadurch ausgefällt werden, daß der Elutionslösung Sulfationen, beispielsweise in Form von Natriumsulfat, zugegeben wird. Die so behandelte Lösung enthält dann praktisch keine zweiwertigen Kationen mehr, sondern das leicht lösliche Natriumchlorid, welches Nachfolgeapplikationen, z.B. enzymatische Applikationen, kaum noch oder gar nicht stört.

Die waschlösung kann einen Alkohol, z.B. Ethanol oder einen anderen kurzkettigen aliphatischen Alkohol, umfassen. Es ist vorteilhaft, eine gepufferte Lösung als Waschlösung und erste Elutionslösung zu verwenden. Der pH-Wert sollte zwischen 4,5 und 9,5 liegen, wobei ein pH-Wert von 7 bevorzugt ist. Der pH-Wert beeinflusst die Ausbeute an doppelsträngiger Nucleinsäure, die im dritten Schritt erzielbar ist, während die Ausbeute an einzelsträngiger Nucleinsäure in der nachfolgenden Reinigung weitgehend unbeeinflusst vom pH-Wert der ersten Elutionslösung ist. Als Puffersubstanz kann jeder geeignete Puffer im fraglichen Pufferbereich verwendet werden. BisTris ist gut geeignet. Eine für das Verfahren nach der Erfindung geeignete Waschlösung kann z.B. folgende Zusammensetzung aufweisen: 10 mM BisTris (pH = 7), 5 mM CaCl₂ und/oder 5 mM MgCl₂, 80 % Ethanol.

Eine für das Verfahren nach der Erfindung geeignete erste Elutionslösung kann z.B. folgende Zusammensetzung aufweisen: 10 mM BisTris (pH = 7), 5 mM CaCl₂ und/oder 5 mM MgCl₂.

Die auf dem Trägermaterial nach dem ersten Elutionsschritt verbliebene einzelsträngigen Nucleinsäure, z.B. RNA, kann mit einem alkoholischen Waschpuffer behandelt werden, der die Erdalkaliionen entfernt. Anschließend wird die RNA unter Niedrigsalzbedingungen oder mit RNase-freiem Wasser eluiert. Auch die so eluierte RNA kann direkt ohne weitere Reinigungsschritte für Folgeapplikationen verwendet werden. Ein Verdau mit DNase ist optional und kann direkt auf dem Trägermaterial vor der Zugabe des alkoholischen Waschpuffers erfolgen. Die Qualität und Quantität der isolierten RNA entspricht der nach Aufreinigung mit speziellen RNA-Isolierungskits. Sie ist weitestgehend frei von genomischer DNA und eignet sich beispielsweise für die RT-PCR.

Die zweite Aufgabe wird erfindungsgemäß durch eine Trennsäule mit einem Trägermaterial, das für die Bindung von einzel- und doppelsträngigen Nucleinsäuren geeignet ist, dadurch gelöst, daß die Trennsäule zumindest ein Erdalkali-Metallsalz in fester Form enthält, das in einem Niedrigsalzpuffer oder Wasser löslich ist und unter Einwirkung dieser Flüssigkeit die zweiwertigen Metallionen in einer mindestens so hohen Konzentration bereitstellt, daß bei der Elution der doppelsträngigen Nucleinsäuren keine einzelsträngigen Nucleinsäuren eluiert werden, wobei die Konzentration an zweiwertigen Metallionen mindestens 1 mM und nicht höher als 200 mM beträgt. Die in fester Form vorliegenden Metallsalze sind unter Einwirkung von Flüssigkeit löslich, so daß die Metallionen sukzessive an die jeweilige Lösung abgeben werden. Die Metallsalze können die schon oben erwähnten Calciumsulfat und/oder Bariumchlorid sein. Der Feststoff ist zweckmäßigerweise dem Trägermaterial zugegeben. Statt dessen kommt jedoch auch die Einlagerung auf einem Filterpapier in Frage.

Die Erfindung wird im Folgenden anhand von Beispielen näher beschrieben und erläutert.

Beispiel 1 : Simultane Isolierung und anschließende Trennung von DNA und RNA aus HeLa-Zellen sowie aus tierischen und pflanzlichen Zellen und Geweben

Für die gleichzeitige Isolierung von DNA und RNA wurde das *NucleoSpin^{®}* RNA II Kit der Fa. MACHEREY-NAGEL mit modifiziertem und erweitertem Protokoll zur Isolierung von RNA verwendet. Im Folgenden wird ein Protokoll für die simultane Isolierung von DNA und RNA aus HeLa-Zellen beschrieben. Anstelle dieser Zellen können jedoch auch Nucleinsäurepräparationen oder Zellen und Gewebeproben unterschiedlichster Herkunft eingesetzt werden. Einige Beispiele sind unten genannt. Anstelle dieses Protokolls können auch andere Protokolle zur Anwendung kommen.

### Lyse und Nualeinsäurobindung an das Trägermaterial

5x10⁶ HeLa Zellen werden durch Zentrifugation geerntet und durch Zugabe von 350 µl (RA1-Lysepuffer aus dem NS RNA II Kit) zum Zellpellet lysiert (3,5 µl β-Mercaptoethanol kann zur Erhöhung des RNA-Schutzes zugegeben werden). Anschließend wird durch Vortexen gemischt. Um das Lysat zu klären, wird durch einen NucleoSpin® Filter zentrifugiert (1 Min., 11.000 g). Anschließend werden die Bindebedingungen durch Zugabe von 350 *µ*l 70 % Ethanol eingestellt. Die Lösung wird durch Vortexen gemischt und auf die NucleoSpin® RNA II Säule gegeben. Anschließend wird für 30 s bei 8.000 g zentrifugiert.

### Waschen der Nucleinsäuren auf der Silicamembran

Auf die Spinsäule mit den gebundenen Nucleinsäuren werden 500 *µ*l Waschlösung gegeben (5 mM MgCl₂, 5 mM CaCl₂, 10 mM BisTris pH 7.0, 80 % Ethanol) und für 1 Min. bei 11.000 g zentrifugiert. Dieser Vorgang wird noch einmal wiederholt. Der Durchlauf wird jeweils verworfen.

### Elution der DNA

Die NucleoSpin® RNA II Säule wird in ein frisches 1,5 ml Auffanggefäß eingesetzt. Anschließend werden 100 *µ*l einer ersten Elutionslösung (5 mM MgCl₂, 5 mM CaCl₂, 10 mM BisTris pH 7.0) zugegeben und die Säule für 1 Min. bei 11.000 g zentrifugiert. Die Temperatur der ersten Elutionslösung sollte 30°C nicht überschreiten, ansonsten kann es zur Co-Elution von RNA kommen. Die eluierte DNA kann direkt für weitere Applikationen wie etwa PCR verwendet werden.

### On-column-Verdau von DNA, Waschen und Elution der RNA

Der DNA-Verdau ist optional. Die DNase-Lösung wird entsprechend dem NucleoSpin® RNA II Protokoll angesetzt und appliziert. Anschließend erfolgen Waschschritte und die Elution der RNA in RNase-freiem Wasser.

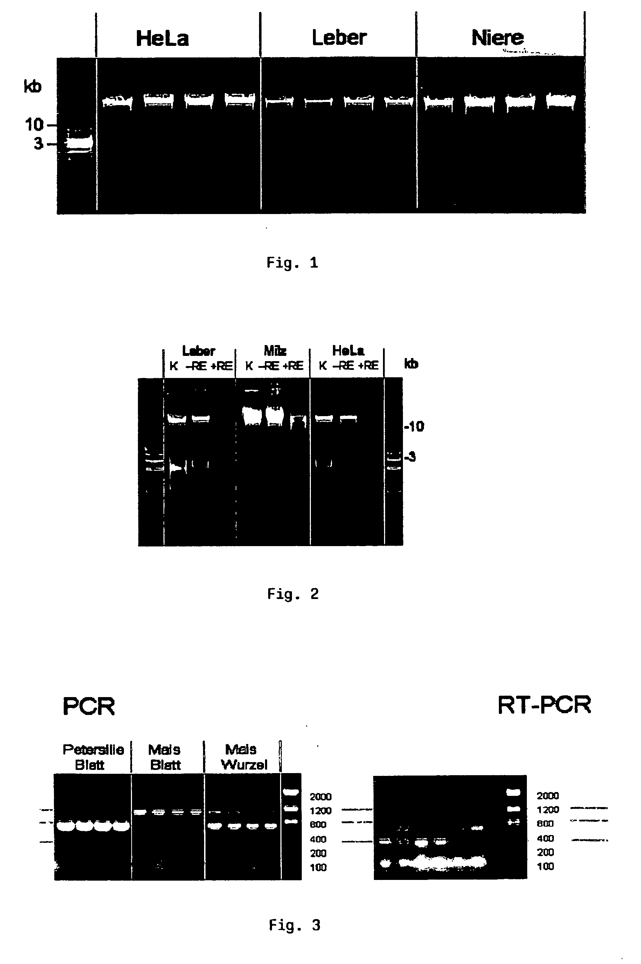

Die Ergebnisse der gleichzeitigen Isolierung und Trennung von DNA und RNA aus verschiedenen Quellen unter Anwendung des obigen Standardprotokolls sind in den Figuren 1-4 dargestellt.
- Figur 1:: Nach dem Standardprotokoll gereinigte DNA aus 1×10⁶ HeLa Zellen sowie jeweils aus 10 mg Schweineleber und Schweineniere. Die DNA wurde in 100 *µ*l eluiert, je 15 *µ*l davon wurden auf das Agarosegel aufgetragen.
- Figur 2:: Restriktionsverdau genomischer DNA, isoliert gemäß Standardprotokoll. Agarose-Gelelektrophorese: Je 1 µg DNA wurde für 1 Std. bei 37°C mit 10 U EcoRI inkubiert. K: Kontrolle ohne EcoRI Restriktion und Inkubation bei 37°C; -RE: Inkubation bei 37°C für 1 Std. ohne EcoRI; +RE: Inkubation für 1 Std. bei 37°C mit EcoRI.
- Figur 3:: Einsatz von nach dem Standardprotokoll isolierter und gereinigter DNA und RNA für PCR und RT-PCR. Aus je 100 mg Pflanzenmaterial wurden nach dem Standardprotokoll DNA und RNA isoliert. Die PCR wurde mit je 2 *µ*l der eluierten DNA als Template, die RT-PCR mit 1 *µ*l der eluierten RNA durchgeführt. Primer nach: Biotechniques 23(3) 1997, pp. 494-498: One primer pair amplifies small subunit ribosomal DNA from miochondria, plastids and bacteria.

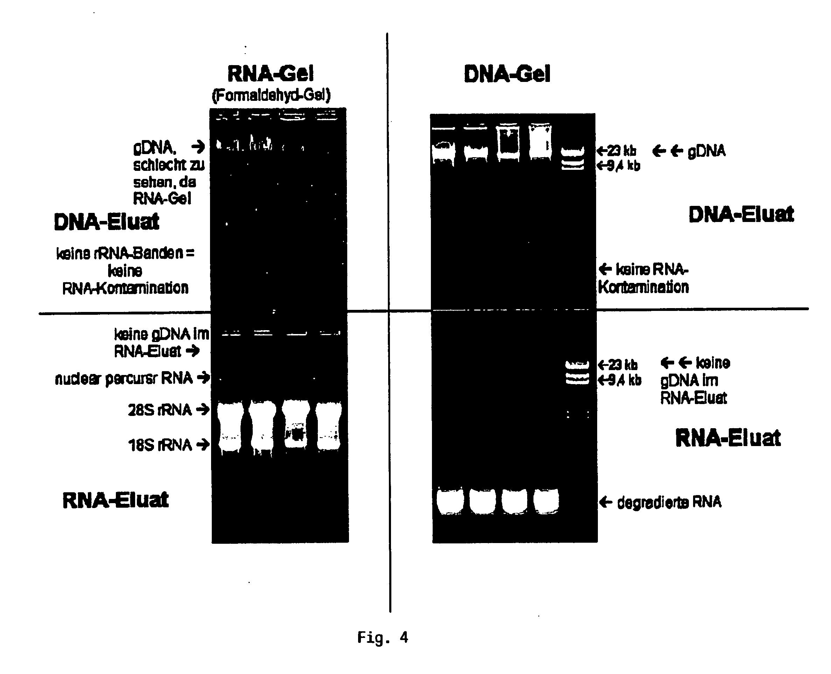

- Figur 4:: Analyse der nach dem Standardprotokoll isolierten und gereinigten DNA und RNA durch Gelelektrophorese. Die linke Seite zeigt ein Formaldehydgel zur Darstellung der RNA und die rechte Seite ein Agarosegel zur Darstellung der DNA. Im oberen Teil der Abbildung wurden Proben der DNA-Eluate aufgetragen und im unteren Teil Proben der RNA-Eluate. Alle Proben, die auf das DNA-Gel geladen wurden, wurden vorab mit RNase behandelt, um die genomische DNA klar zu visualisieren.

Wie Figur 1 deutlich zeigt, wurde sowohl aus HeLa-Zellen als auch aus zwei weiteren Geweben hochmolekulare (> 20 kbp) genomische DNA isoliert. Das A_{260/280} Verhältnis lag in allen Fällen über 2,0.

Die Eignung der eluierten DNA für Folgeapplikationen ist in Figur 2 dargestellt. Die aus drei verschiedenen Probenmaterialien isolierte DNA wurde hier einem Restriktionsverdau mit EcoRI unterzogen. Wie der Vergleich K/-RE zeigt, ist die isolierte DNA für 1 Std. bei 37°C stabil, was für die Integrität und Reinheit der DNA spricht. Der Vergleich -RE/+RE zeigt, dass die DNA restringierbar ist.

Die Eignung von isolierter DNA und RNA für Folgeapplikationen, z.B. PCR, ist auch Figur 3 zu entnehmen. In der Abbildung links sind die für die Primer beschriebenen typischen Banden bei ca. 750 und 1200 bp deutlich sichtbar. Als, Probenmaterial wurden drei verschiedene Pflanzengewebe eingesetzt. Figur 3 zeigt zudem die Eignung der isolierten RNA für die wichtige Nachfolgeapplikation der RT-PCR (vgl. Figur 3, rechts). RNA, die nach dem erfindungsgemäßen Verfahren isoliert wurde, wurde durch reverse Transkription in DNA umgeschrieben und diese anschließend mittels PCR amplifiziert. Die Figur 3 rechts zeigt hier die typischen Bandenmuster und belegt somit die Eignung der eingesetzten RNA für die RT-PCR.

Gelelektrophoretieche Analysen der isolierten DNA und RNA sind in Figur 4 dargestellt. Das DNA-Eluat auf dem RNA-Gel zeigt keine Banden für ribosomale RNA, was belegt, dass die DNA weitestgehend RNA-frei ist. Die genomische DNA selbst ist auf dem für RNA ausgelegten Formaldehydgel nur schlecht zu erkennen. Die gleiche Probe auf dem DNA-Gel zeigt hingegen deutlich ein Bande - 20 kbp für intakte genomische DNA. RNA-Kontaminationen sind auch hier nicht zu erkennen. Die Banden für 18S und 28S ribosomale RNA sind auf dem RNA-Gel und unter Einsatz der RNA-Eluate deutlich zu sehen. In diesem Fall fehlt jeder Hinweis auf DNA-Kontaminationen, der auch auf dem DNA-Gel der RNA-Eluate gänzlich ausbleibt. Das DNA-Gel zeigt hingegen nur die degradierte RNA (um DNA besser zu visualisieren, wurde hier ein RNase-Verdau durchgeführt).

### Beispiel 2: Ermittlung des goeigneten pH-Bereiches für die erste Elutionslösung

Probenmaterial : Je Präparation ca. 10⁶ HeLa-Zellen. Isolierung der Nucleinsäuren gemäß Standardprotokoll von Beispiel 1. Die Waschlösung war bei allen Experimenten die folgende: 10 mM BisTris pH = 7; 5 mM CaCl₂;5' mM MgCl₂; 80% Ethanol. Bei der Elution der DNA wurden die folgenden Varianten für die 1. Elutionslösung getestet:

| | | | | | | | | | | | |
|---|---|---|---|---|---|---|---|---|---|---|---|
| A. | 10 | mM | Ameisensäure-NaOH, | pH = | 4 | 5 | mM | CaCl₂; | 5 | mM | MgCl₂ |
| B. | 10 | mM | Essigsäure-NaOH, | pH = | 5 | 5 | mM | CaCl₂; | 5 | mM | MgCl₂ |
| C. | 10 | mM | BisTris, | pH = | 6 | 5 | mM | CaCl₂; | 5 | mM | MgCl₂ |
| D. | 10 | mM | BisTris, | pH = | 7 | 5 | mM | CaCl₂; | 5 | mM | MgCl₂ |
| E. | 10 | mM | Tris, | pH = | 9 | 5 | mM | CaCl₂; | 5 | mM | MgCl₂ |
| F. | 10 | mM | Ethanolamin, | pH = | 9,5 | 5 | mM | CaCl₂; | 5 | mM | MgCl₂ |
| G. | 10 | mM | Ethanolamin, | pH = | 10 | 5 | mM | CaCl₂; | 5 | mM | MgCl₂ |

Es wurden Doppelbestimmungen durchgeführt. Die Ergebnisse sind im folgenden Diagramm dargestellt.

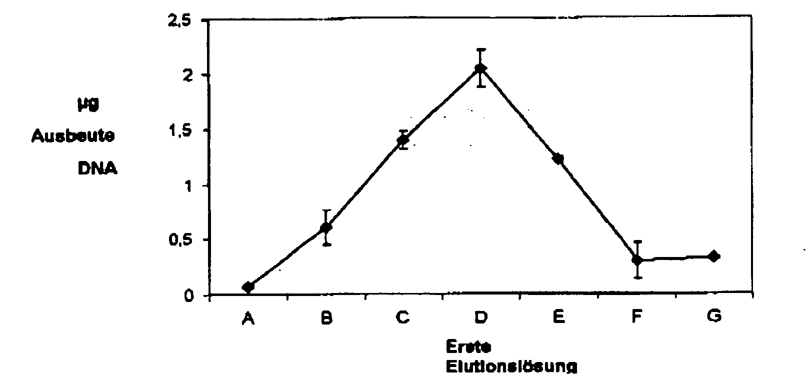

Die DNA-Ausbeute hängt gemäß obigem Diagram deutlich vom pH-Wert der eingesetzten Lösung ab und folgt einer typischen Optimumskurve. Der bevorzugte pH-Wert liegt bei 7,0.

Die RNA-Ausbeute in der nachfolgenden Reinigung blieb weitgehend unbeeinflusst von dem pH-Wert der obigen Elutionslösungen, wobei eine gewisse Beeinträchtigung der RNA-Qualität bei pH-Werten der 1. Elutionslösung über 8 zu erkennen war (Analyse über Gelelektrophorese).

Die obigen Ergebnisse des Beispiels 2 deuten auch an, dass mit verschiedenen Puffersubstanzen gearbeitet werden kann. Entscheidend ist der pH-Wert.

### Beispiel 3: Effekt von Ca/MqCl₂ auf die Nucleinsäureausbeute

ziel dieser Versuchsreihe war es, den Einfluss der An- und Abwesenheit von Ca/MgCl₂ in der Waschlösung auf die notwendige Konzentration der zweiwertigen Ionen in der 1. Elutionslösung zu untersuchen. Das Probenmaterial war je eine Präparation von ca. 10⁶ HeLa-Zellen. Isolierung der Nucleinsäuren gemäß Standardprotokoll von Beispiel 1.

Bei Experiment 1 wurde eine Waschlösung mit zweiwertigen Metallionen verwendet: 10 mM BisTris (pH = 7), 5 mM CaCl_{2'} 5 mM MgCl₂, 80% Ethanol. Bei Experiment 2 wurde eine Waschlösung ohne zweiwertige Metallionen verwendet: 10 mM BisTris (pH = 7), 80% Ethanol.

Bei Experiment 1 und 2 wurden die folgenden 9 Elutionslösungen als erste Elutionslösung eingesetzt:

| | | | | | | | |
|---|---|---|---|---|---|---|---|
| A. | 10 | mM | BisTris | pH = | 7 | kein | Salzzusatz |
| B. | 10 | mM | BisTris | pH = | 7 | 1 mM | CaCl₂ |
| C. | 10 | mM | BisTris | pH = | 7 | 5 mM | CaCl₂ |
| D. | 10 | mM | BisTris | pH = | 7 | 10 mM | CaCl₂ |
| E. | 10 | mM | BisTris | pH = | 7 | 100 mM | CaCl₂ |
| F. | 10 | mM | BisTris | pH = | 7 | 1 mM | MgCl₂ |
| G. | 10 | mM | BisTris | pH = | 7 | 5 mM | MgCl₂ |
| H. | 10 | mM | BisTris | pH = | 7 | 10 | mM MgCl₂ |
| I. | 10 | mM | BisTris | pH = | 7 | 100 | mM MgCl₂ |

Insgesamt wurden 18 Präparationen je Experiment (Doppelpräparationen unter Verwendung der 9 verschiedenen Elutionslösungen) getestet. Die Ergebnisse von Experiment 1 sind im folgenden Diagramm dargestellt:
**Experiment 1:** Waschlösung mit Metallionen. Dunkle Balken: Nukleinsäuren im ersten Eluat; Helle Balken: Nukleinsäuren im zweiten Eluat, d.h. Nukleinsäuren, die nach Behandlung mit der ersten Elutionslösung auf der Säule verblieben. 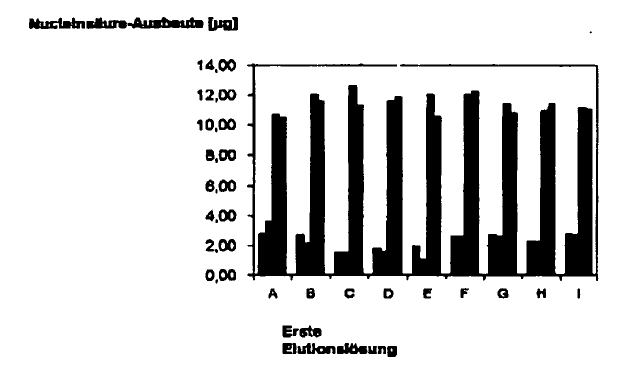
Nucleinsäure-Ausbeute [µg]

14,00
12,00
10,00
8,00
6,00
4,00
2,00
0,00


A B C D E F G H I

Erste
Elutionslösung
 Bei Anwesenheit von Ca/MgCl₂ in der Waschlösung lässt sich im nachfolgenden Schritt mit allen getesteten Varianten der erste Elutionslösung DNA von der Säule waschen, ohne dass diese nach TAE und Formaldehyd-Gelelektrophorese offensichtlich mit RNA kontaminiert ist. Dies gilt insbesondere auch für die erste Elutionslösung A, die keine zweiwertigen Metallionen enthielt. Dies bedeutet, dass selbst eine Elution der DNA mit 10 mM BisTris möglich war unter gleichzeitigem Verbleib von RNA auf der Säule. Eine möglichst niedrige Ionenkonzentration ist für die Nachfolgeapplikationen sehr wichtig. Als Erklärung für den Verbleib der RNA auf der Säule ist anzunehmen, dass die durch die Waschlösung auf die Säule und RNA/DNA übertragene Salzmenge ausreicht, um bei Zugabe einer 1. Elutionslösung ohne zweiwertige Metallionen die RNA auf der Säule zu halten. Die Analyse mit Gelelektrophorese zeigt, dass die Nucleinsäuren in der 1. Elutionslösung praktisch nur aus DNA bestanden und RNA nicht mit eluiert wurde. Die Nucleinsäuren in finalen Eluat zeigten hingegen ausschließlich RNA.
   Das folgende Diagram zeigt die Ergebnisse von Experiment 2.
**Experiment 2:** Waschlösung ohne Metallionen. Dunkle Balken: Nukleinsäuren im ersten Eluat; Helle Balken: Nukleinsäuren im zweiten Eluat, d.h. Nukleinsäuren, die die nach Behandlung mit der ersten Elutionslösung auf der Säule verblieben. 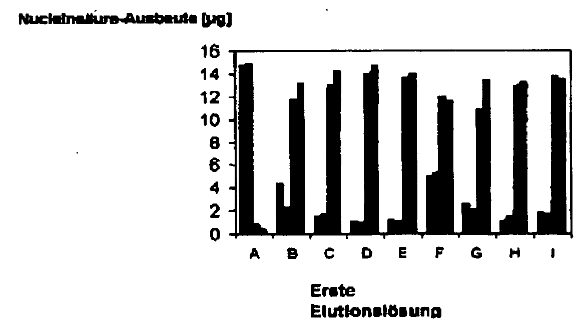
Nucleinsäure-Ausbeute [µg]
16
14
12
10
8
6
4
2
0
A B C D E F G H I
Erste
Elutionslösung
 Bei Abwesenheit von Ca/MgCl₂ in der Waschlösung führt die nachfolgende Aufgabe von 100 µl der ionenfreien ersten Elutionslösung A (10 mM BisTris pH 7) zur Elution von DNA und RNA, da diese Elution in Abwesenheit von zweiwertigen Metallionen durchgeführt wurde. Die Gegenwart von 1 bis 100 mM CaCl₂ oder 1 bis 100 mM NgCl₂ in der ersten Elutionslösung ermöglichte eine DNA-Elution unter Verbleib der RNA auf der Säule. Die RNA wurde dann im zweiten Eluat gefunden.

Die Ergebnisse der Experimente 1 und 2 lassen sich wie folgt zusammenfassen: Wird eine Waschlösung ohne zweiwertige Ionen, z.B. Mg²⁺/Ca²⁺, eingesetzt, muss die erste Elutionslösung mindestens 1 mM CaCl₂ oder MgCl₂ enthalten. Die bevorzugte Ausführung sieht jeweils 5 mM vor. Um Nachfolgeapplikationen nicht zu stören, sind möglichst geringe Konzentrationen empfehlenswert. Sind dagegen zweiwertige Ionen bereits in der Waschlösung vorhanden, kann die erste Elutionslösung auch frei von zweiwertigen Metallionen sein. Das Beispiel 3 zeigt ferner, dass die Anwesenheit eines Metallione (Ca²⁺ oder Mg²⁺) ausreichend ist, um die RNA auf der Säule zu halten. Die Ergebnisse von Beispiel 2 belegen, dass auch 2 verschiedene Metallionen sowohl in der Waachlösung als auch in der erste Elutionslösung verwendet werden können.

### Beispiel 4: Test verschiedener Metallionen

In dieser Versuchsreihe wurden verschiedene Metallionen eingesetzt. Die Ionen waren sowohl in der waschlösung als auch in der ersten Elutionslösung vorhanden:

| Stock # | Salz |
|---|---|
| 1 | MgCl₂ x 6 H₂O |
| 2 | CaCl₂ (wasserfrei) |
| 3 | BaCl₂ x 2 H₂O |
| 4 | NaCl |
| 5 | MnCl₂ |
| 6 | ZnCl₂ |
| 7 | CaI₂ x H₂O |
| 8 | MgSO₄ |
| 9 | CuSO₄ x 5 H₂O |
| 10 | Licl |
| 11 | CuCl₂ |
| 12 | BeSO₄ x 4 H₂O |
| 13 | SrCl₂ x 6 H₂O |

Varianten der Waschlösung 1-13: Jeweils 10 mM BisTris, pH 7; 5 mM Salz (#1-13); 80% Ethanol. Varianten der ersten Elutionslösung 1-13: Jeweils 10 mM BisTris, pH 7; 5 mM Salz (#1-13). Es wurden 13 Lösungspaare (waschlösung/erste Elutionslösung) jeweils im Doppelpräp. --> 26 Präps eingesetzt. Je Präp. 2 Eluate (DNA-Eluat/RNA-Eluat) --> insgesamt 52 Eluate.

### Protokoll:

- HeLa-Zellen in 350 µl RA1 aufnehmen, 10⁶ HeLa-Zellen je Präp.
- Je 350 µl Ethanol dazu, mixen.
- Auftrag auf NS RNA II-Säule.
- + 500 µl Waschlösung #1-13 , zentrifugieren für 1 Min. bei 11000xg
- + 500 *µ*l Waschlösung #1-13, zentrifugieren für 1 Min. bei 11000xg, hiernach Säule in frisches Eppi stellen.
- + 100 *µ*l erste Elutionslösung (Lösungen #1-13), zentrifugieren für 1 Min. bei 11000xg (DNA-Eluat)
- Weiter mit Schritt 7 Seite 15 (*Digest DNA*) des NS RNA II Handbuches Version May 2003, Rev. 02.
- Finale Elution der RNA mit 100 *µ*l Wasser (RNA-Eluat) Quantifizierung mittels A260, 1 % TAE Gelelektrophorese (DNA) und 1,2 % denaturierendes Formaldehydgel (RNA). Die Ergebnisse sind in der folgenden Tabelle dargestellt. Aufgeführt sind alle getesteten Metallionen mit den verwendeten Gegenionen.

| DNA-Elution | | | |
|---|---|---|---|
| | Cl ⁻ | I ⁻ | SO₄ ²⁻ |
| Li⁺ | enthält auch RNA | | |
| Na⁺ | enthält auch RNA | | |
| Be ²⁺ | | | keine |
| Mg ²⁺ | sehr gut | | sehr gut |
| Ca ²⁺ | mäßig | mäßig | |
| Sr ²⁺ | sehr gut | | |
| Ba ²⁺ | mäßig | | |
| Mn ²⁺ | kaum | | |
| Cu ²⁺ | keine | | keine |
| Zn ²⁺ | keine | | |

| RNA-Elution | | | |
|---|---|---|---|
| | Cl ⁻ | I ⁻ | SO₄ ²⁻ |
| Li⁺ | kaum, da schon mit DNA eluiert | | |
| Na⁺ | kaum, da schon mit DNA eluiert | | |
| Be ²⁺ | | | kaum |
| Mg ²⁺ | sehr gut | | sehr gut |
| Ca ²⁺ | sehr gut | sehr gut | |
| Sr ²⁺ | gut | | |
| Ba ²⁺ | sehr gut | | |
| Mn ²⁺ | sehr gut | | |
| Cu ²⁺ | gut | | sehr gut, aber mit DNA kontaminiert |
| Zn ²⁺ | sehr gut, aber deutlich mit DNA kontaminier t | | |

| Gesamtbeurteilung für DNA- und RNA-Elution | | | |
|---|---|---|---|
| | Cl⁻ | I⁻ | SO₄²⁻ |
| Li⁺ | RNA in DNA-Eluat | | |
| Na⁺ | RNA in DNA-Eluat | | |
| Be ²⁺ | | | keine DNA, kaum RNA |
| Mg ²⁺ | sehr gut | | sehr gut |
| Ca ²⁺ | weniger DNA als mit Mg | weniger DNA als mit Mg | |
| Sr ²⁺ | weniger RNA ale mit Mg | | |
| Ba ²⁺ | wenig DNA | | |
| Mn ²⁺ | kaum DNA | | |
| Cu ²⁺ | keine DNA | | keine DNA |
| Zn ²⁺ | keine DNA | | |

Wie man aus der Gesamtbeurteilung erkennt, sind unter den hier gewählten Bedingungen insbesondere Magnesium und Calcium, aber auch Strontium und Barium geeignet. Die Gegenionen spielen nur eine untergeordnete Rolle, wie man am Beispiel des Chloride (einige Metallionen sehr gut, einige sehr schlecht) sieht. Ähnliches gilt für das Sulfat (Magnesium sehr gut, Kupfer sehr schlecht). Auch wenn die meisten Experimente mit Cl⁻ gemacht wurden, zeigen die Daten, dass auch andere Gegenionen in gleicher Weise zu verwenden sind. Einwertige Metallionen (siehe Lithium, Natrium) sind dagegen nicht geeignet.

## Patentansprüche

1. Verfahren zur Isolierung und Trennung von einzel- und doppelsträngigen Nucleinsäuren aus einer diese Nucleinsäuren enthaltenden Probe, wobei die doppelsträngige Nucleinsäure DNA und die einzelsträngige Nucleinsäure RNA ist und die einzel- und doppelsträngigen Nucleinsäuren an ein Trägermaterial gebunden werden, von dem die doppelsträngigen Nucleinsäuren mit einer ersten Elutionslösung und die einzelsträngigen Nucleinsäuren anschließend mit einer zweiten Elutionslösung eluiert werden, **dadurch gekennzeichnet, dass** die Elution der doppelsträngigen Nucleinsäuren in Gegenwart von mindestens einem solchen zweiwertigen Metallion in einer wenigstens so hohen Konzentration durchgeführt wird, daß bei der Elution der doppelsträngigen Nucleinsäuren keine einzelsträngigen Nucleinsäuren eluiert werden, wobei die erste Elutionslösung ein Niedrigsalzpuffer oder Wasser und die zweite Elutionslösung eine Niedrigsalzlösung oder Wasser ist und wobei das mindestens eine zweiwertige Metallion aus der Gruppe der Erdalkalimetalle ausgewählt wird und dessen Konzentration nicht höher als 200 mM ist.

2. Verfahren nach Anspruch 1, **dadurch gekennzeichnet, daß** die erste Elutionslösung das mindestens eine zweiwertige Metallion mit einer Konzentration von mindestens 1 mM umfasst.

3. Verfahren nach Anspruch 1 oder 2, **dadurch gekennzeichnet, daß** das Trägermaterial nach der Bindung der Nucleinsäuren und vor der Eluierung der doppelsträngigen Nucleinsäuren mit einer waschlösung behandelt wird.

4. Verfahren nach Anspruch 3, **dadurch gekennzeichnet, daß** die Waschlösung das mindestens eine zweiwertige Metallion enthält.

5. Verfahren nach Anspruch 3 oder 4, **dadurch gekennzeichnet, daß** die Waschlösung einen Alkohol, vorzugsweise einen aliphatischen Alkohol mit 1 bis 5 Kohlenstoffatomen, umfasst.

6. Verfahren nach einem der Ansprüche 3 bis 5, **dadurch gekennzeichnet, daß** die Waschlösung eine Puffersubstanz umfasst und der pH-Wert der Waschlösung zwischen 4,5 und 9,5 eingestellt wird.

7. Verfahren nach Anspruch 6, **dadurch gekennzeichnet, daß** der pH-Wert der Waschlösung auf 7 eingestellt wird.

8. Verfahren nach einem der Ansprüche 1 bis 7, **dadurch gekennzeichnet, daß** das mindestens eine zweiwertige Metallion bei der Elution der doppelsträngigen Nucleinsäuren in einer Konzentration von höchstens 100 mM vorliegt.

9. Verfahren nach einem der Ansprüche 1 bis 8, **dadurch gekennzeichnet, daß** das mindestens eine zweiwertige Metallion bei der Elution der doppelsträngigen Nucleinsäuren in einer Konzentration von höchstens 20 mM vorliegt.

10. Verfahren nach einem der Ansprüche 1 bis 9, **dadurch gekennzeichnet, daß** das mindestens eine zweiwertige Metallion ein Calciumion, Magnesiumion, Bariumion und/oder Strontiumion ist.

11. Verfahren nach Anspruch 10, **dadurch gekennzeichnet, daß** Calciumionen in Form von Kalziumsulfat eingebracht werden.

12. Verfahren nach Anspruch 10 oder 11, **dadurch gekennzeichnet, daß** Bariumionen in Form von Bariumsalzen eingebracht werden.

13. Verfahren nach Anspruch 11 oder 12, **dadurch gekennzeichnet, daß** das Eluat mit den doppelsträngigen Nucleinsäuren mit Sulfationen derart behandelt wird, daß im Eluat noch enthaltene Kationen ausgefällt werden.

14. Verfahren nach einem der Ansprüche 1 bis 13, **dadurch gekennzeichnet, daß** die erste Elutionslösung eine Puffersubstanz umfasst und der pH-Wert der ersten Elutionslösung zwischen 4,5 und 9,5 eingestellt wird.

15. Verfahren nach Anspruch 14, **dadurch gekennzeichnet, daß** der pH-Wert der ersten Elutionslösung auf 7 eingestellt wird.

16. Verfahren nach einem der Ansprüche 1 bis 15, **dadurch gekennzeichnet, daß** nach der Elution der doppelsträngigen Nucleinsäure und vor der Elution der einzelsträngigen Nucleinsäure ein DNA-Verdau möglicher Rest-DNA durchgeführt wird.

17. Verfahren nach einem der Ansprüche 1 bis 16, **dadurch gekennzeichnet, daß** nach Elution der doppelsträngigen Nucleinsäuren und vor Elution der einzelsträngigen Nucleinsäuren das Trägermaterial mit einem alkoholischen Waschpuffer gewaschen wird.

18. Trennsäule zur Durchführung des Verfahrens nach einem der Ansprüche 1 bis 17, mit einem Trägermaterial, das für die Bindung von einzel- und doppelsträngigen Nucleinsäuren geeignet ist, **dadurch gekennzeichnet, daß** die Trennsäule zumindest ein Erdalkali-Metallsalz in fester Form enthält, das in einem Niedrigsalzpuffer oder Wasser löslich ist und unter Einwirkung dieser Flüssigkeit die zweiwertigen Metallionen in einer mindestens so hohen Konzentration bereitstellt, daß bei der Elution der doppelsträngigen Nucleinsäuren keine einzelsträngigen Nucleinsäuren eluiert werden, wobei die Konzentration an zweiwertigen Metallionen mindestens 1 mM und nicht höher als 200 mM beträgt.

19. Trennsäule nach Anspruch 18, **dadurch gekennzeichnet, daß** die Metallsalze Calciumsulfat und/oder Bariumchlorid ist bzw. sind.

20. Trennsäule nach Anspruch 18 oder 19, **dadurch gekennzeichnet, daß** der Feststoff dem Trägermaterial zugeordnet ist.

21. Trennsäule nach einem der Ansprüche 18 bis 20, **dadurch gekennzeichnet, daß** der Feststoff an einem Filterpapier vorliegt.

## Claims

1. A process for isolating and separating single- and double-stranded nucleic acids from a specimen containing these nucleic acids, the double-stranded nucleic acid being DNA, and the single-stranded nucleic acid being RNA, and the single- and double-stranded nucleic acids being bonded to a carrier material of which the double-stranded nucleic acids are eluted with a first elution solution, and the single-stranded nucleic acids are then eluted with a second elution solution, **characterised in that** the elution of the double-stranded nucleic acids is implemented in the presence of at least one such bivalent metal ion in a concentration which is at least so high that with the elution of the double-stranded nucleic acids no single-stranded nucleic acids are eluted, the first elution solution being a low salt buffer or water, and the second elution solution being a low salt solution or water, and the at least one bivalent metal iron being selected from the group of the alkaline earth metals, and the concentration of the latter being no higher than 200 mM.

2. The process according to Claim 1, **characterised in that** the first elution solution comprises the at least one bivalent metal iron with a concentration of at least 1 mM.

3. The process according to Claim 1 or 2, **characterised in that** after the binding of the nucleic acids and before the elution of the double-stranded nucleic acids the carrier material is treated with a washing solution.

4. The process according to Claim 3, **characterised in that** the washing solution contains the at least one bivalent metal ion.

5. The process according to Claim 3 or 4, **characterised in that** the washing solution comprises an alcohol, preferably an aliphatic alcohol with 1 to 5 carbon atoms.

6. The process according to any of Claims 3 to 5, **characterised in that** the washing solution comprises a buffer substance, and the pH value of the washing solution is set at between 4.5 and 9.5.

7. The process according to Claim 6, **characterised in that** the pH value of the washing solution is set at 7.

8. The process according to any of Claims 1 to 7, **characterised in that** during the elution of the double-stranded nucleic acids the at least one bivalent metal ion is in a concentration of maximum 100 mM.

9. The process according to any of Claims 1 to 8, **characterised in that** during the elution of the double-stranded nucleic acids the at least one bivalent metal ion is present in a concentration of maximum 20 mM.

10. The process according to any of Claims 1 to 9, **characterised in that** the at least one bivalent metal ion is a calcium ion, magnesium ion, barium ion and/or strontium ion.

11. The process according to Claim 10, **characterised in that** calcium ions are introduced in the form of calcium sulphate.

12. The process according to Claim 10 or 11, **characterised in that** barium ions are introduced in the form of barium salts.

13. The process according to Claim 11 or 12, **characterised in that** the eluate with the double-stranded nucleic acids is treated with sulphate ions such that cations still contained in the eluate are eliminated.

14. The process according to any of Claims 1 to 13, **characterised in that** the first elution solution comprises a buffer substance, and the pH value of the first elution solution is set at between 4.5 and 9.5.

15. The process according to Claim 14, **characterised in that** the pH value of the first elution solution is set at 7.

16. The process according to any of Claims 1 to 15, **characterised in that** after the elution of the double-stranded nucleic acid and before the elution of the single-stranded nucleic acid a DNA digestion of any remaining DNA is implemented.

17. The process according to any of Claims 1 to 16, **characterised in that** after elution of the double-stranded nucleic acids and before elution of the single-stranded nucleic acids the carrier material is washed with an alcoholic washing buffer.

18. A separation column for implementing the process according to any of Claims 1 to 17, comprising a carrier material which is suitable for the binding of single- and double-stranded nucleic acids, **characterised in that** the separation column contains at least one alkaline earth metal salt in solid form, which is soluble in a low salt buffer or water, and under the effect of this liquid prepares the bivalent metal ions in a concentration which is at least so high that with the elution of the double-stranded nucleic acids no single-stranded nucleic acids are eluted, the concentration of bivalent metal ions being at least 1 mM and no higher than 200 mM.

19. The separation column according to Claim 18, **characterised in that** the metal salts are calcium sulphate and/or barium chloride.

20. The separation column according to Claim 18 or 19, **characterised in that** the solid is assigned to the carrier material.

21. The separation column according to any of Claims 18 to 20, **characterised in that** the solid is present on a filter paper.

## Revendications

1. Procédé pour l'isolation et la séparation d' acides nucléiques à chaîne simple et double issus d'un échantillon contenant ces acides nucléiques, sachant que l'acide nucléique à chaîne double est ADN et l'acide nucléique à chaîne simple est ARN et les acides nucléiques à chaîne simple et à chaîne double sont liés à un matériau porteur, à partir duquel les acides nucléiques à chaîne double sont élués avec une première solution d'élution et les acides nucléiques à chaîne simple sont ensuite élués avec une deuxième solution d' élution, **caractérisé en ce que** l'élution des acides nucléiques à chaîne double est exécutée en présence d'au moins un tel ion métallique bivalent dans une concentration au moins élevée de manière à ce que lors de l'élution des acides nucléiques à chaîne double, aucun acide nucléique à chaîne simple ne soit élué, sachant que la première solution d'élution est un tampon à faible teneur en sel ou de l'eau et la deuxième solution d'élution est une solution à faible teneur en sel ou de l'eau et sachant que l'ion métallique bivalent minimum est sélectionné dans le groupe des métaux alcalino-terreux et dont la concentration n'est pas supérieure à 200 mM.

2. Procédé selon revendication 1, **caractérisé en ce que** la première solution d'élution comprend l'ion métallique bivalent minimum avec une concentration d'au moins 1 mM.

3. Procédé selon revendication 1 ou 2, **caractérisé en ce que** le matériau porteur est traité avec une solution de lavage après la fixation des acides nucléiques et avant l'élution des acides nucléiques à chaîne double.

4. Procédé selon revendication 3, **caractérisé en ce que** la solution de lavage contient l'ion métallique bivalent minimum.

5. Procédé selon revendication 3, **caractérisé en ce que** la solution de lavage comprend un alcool, de préférence un alcool aliphatique avec 1 à 5 atomes de carbone.

6. Procédé selon l'une des revendications 3 à 5, **caractérisé en ce que** la solution de lavage comprend une substance tampon et la valeur de pH de la solution de lavage est ajustée entre 4,5 et 9,5.

7. Procédé selon revendication 6, **caractérisé en ce que** la valeur de pH de la solution de lavage est ajustée à 7.

8. Procédé selon l'une des revendications 1 à 7, **caractérisé en ce que** l'ion métallique bivalent minimum existe lors de l'élution des acides nucléiques à chaîne double dans une concentration de 10 mM maximum.

9. Procédé selon l'une des revendications 1 à 8, **caractérisé en ce que** l'ion métallique bivalent minimum existe lors de l'élution des acides nucléiques à chaîne double dans une concentration de 20 mM maximum.

10. Procédé selon l'une des revendications 1 à 9, **caractérisé en ce que** l'ion métallique bivalent minimum est un ion de calcium, ion de magnésium, ion de baryum et / ou ion de strontium.

11. Procédé selon revendication 10, **caractérisé en ce que** des ions de calcium sont apportés sous forme de sulfate de calcium.

12. Procédé selon revendication 10 ou 11, **caractérisé en ce que** des ions de baryum sont apportés sous forme de sels de baryum.

13. Procédé selon revendication 11 ou 12, **caractérisé en ce que** l'éluât est traité avec les acides nucléiques à chaîne double avec des ions de sulfate, de telle manière que dans l'éluât, des cations encore contenus soient précipités

14. Procédé selon l'une des revendications 1 à 13, **caractérisé en ce que** la première solution d'élution comprend une substance tampon et la valeur de pH de la première solution d'élution est ajustée entre 4,5 et 9,5.

15. Procédé selon revendication 14, **caractérisé en ce que** la valeur de pH de la première solution d'élution est ajustée à 7.

16. Procédé selon l'une des revendications 1 à 15, **caractérisé en ce qu'**après l'élution de l'acide nucléique à chaîne double et avant l'élution de l'acide nucléique à chaîne simple, une digestion d'ADN d'un reste éventuel d'ADN s'effectue.

17. Procédé selon l'une des revendications 1 à 16, **caractérisé en ce qu'**après l'élution des acides nucléiques à chaîne double, et avant l'élution des acides nucléiques à chaîne simple, le matériau porteur est lavé avec un tampon de lavage alcoolisé.

18. Colonne de rectification pour la réalisation du procédé selon l'une des revendications 1 à 17, avec un matériau porteur qui est approprié pour la fixation d'acides nucléiques à chaîne simple ou double, **caractérisée en ce que** la colonne de rectification contient au moins un sel métallique alcalino-terreux sous forme solide, qui est soluble dans un tampon à faible teneur en sel ou dans de l'eau et sous l'effet de ce liquide, fournit des ions métalliques bivalents dans une concentration au moins élevée de telle manière que lors de l'élution des acides nucléiques à chaîne double, aucun acide nucléique à chaîne simple ne soit élué, sachant que la concentration en ions métalliques bivalents est d'au moins 1 nM et non supérieure à 200 mM.

19. Colonne de rectification selon revendication 18, **caractérisée en ce que** les sels métalliques sont du sulfate de calcium et / ou chlorure de baryum, etc.

20. Colonne de rectification selon revendication 18 ou 19, **caractérisée en ce que** la matière solide est assignée au matériau porteur.

21. Colonne de rectification selon l'une des revendications 18 à 20, **caractérisée en ce que** la matière solide est disponible sur un papier-filtre.
